(19) Europäisches Patentamt European Patent Office Office européen des brevets

(11) EP 1 743 664 B1

(12) EUROPEAN PATENT SPECIFICATION

(45) Date of publication and mention
of the grant of the patent:
**16.12.2009 Bulletin 2009/51**

(51) Int Cl.:
*A61L 29/12* (2006.01)          *B29C 47/00* (2006.01)
*A61L 29/14* (2006.01)          *A61M 25/10* (2006.01)

(21) Application number: **06017985.0**

(22) Date of filing: **25.10.2000**

(54) **Dimensionally stable balloons**

formstabile Ballons

Ballonnets aux dimensions stables

(84) Designated Contracting States:
**AT BE CH CY DE DK ES FI FR GB GR IE IT LI LU
MC NL PT SE**

(30) Priority: **25.10.1999 US 426384**

(43) Date of publication of application:
**17.01.2007 Bulletin 2007/03**

(62) Document number(s) of the earlier application(s) in
accordance with Art. 76 EPC:
**00989702.6 / 1 223 992**

(73) Proprietor: **Boston Scientific Limited
St. Michael, Barbados, West Indies (BB)**

(72) Inventors:
• **Chen, John, Jianhua
Plymouth, MN 55446 (US)**
• **Wang, Lixiao
Maple Grove, MN 55369 (US)**
• **Wang, Yiqun
Maple Grove, MN 55311 (US)**
• **Chin, Albert, C.C.
Newton, MA 02461 (US)**

(74) Representative: **Hauck Patent- und Rechtsanwälte
Neuer Wall 50
20354 Hamburg (DE)**

(56) References cited:
**EP-A- 0 934 755      WO-A-00/50105
US-A- 5 248 305**

## Description

BACKGROUND OF THE INVENTION

Medical catheters having a balloon mounted thereon are useful in a variety of medical procedures. A balloon may be used to widen a vessel into which the catheter is inserted by dilating the blocked vessel, such as in an angioplasty procedure. More significant to the present invention however, is the use of a catheter to deliver a medical device, such as a stent, into a body lumen. Some examples of stent delivery balloons are disclosed in U.S. Patent No. 5702418, and U.S. Patent No. 5,797,877 , In these and other medical device delivery applications, radial expansion of a balloon may be used to expand or inflate a stent at a desired positioned within the body. Using a balloon equipped catheter to deliver a stent requires precise positioning of the balloon and stent as well as a balloon with accurate and predictable expansion properties. A known drawback of many previous delivery catheters and balloons is that when a balloon is radially inflated to a desired extent, the balloon will also expand longitudinally. As a result of longitudinal expansion of a balloon during the delivery of a medical device, the balloon itself, the medical device mounted thereupon or both apparatuses may be shifted from their pre-inflation position resulting in improper delivery of the medical device.

In balloons where longitudinal expansion occurs, the balloon may expand longitudinally past one or both of the stent ends. Typical stent delivery balloons will expand longitudinally at least 5% beyond the original pre-inflation state. In addition to potentially mis-delivering the medical device as described above, the resulting extended balloon may cause the edges of the stent to push against the vessel wall to a greater extent than they would from radial expansion alone. The protruding stent edges may damage or tear the surrounding vessel resulting in potentially serious trauma for the patient.

It has recently been discovered that Liquid Crystal Polymers (LCP) may be effectively blended with other materials and extruded to form high strength medical balloons. In copending U.S. applications 08/926,905 (corresponding to PCT/US98/18345 filed Sept. 4, 1998 ), and 09/257,677 filed February 25, 1999 there are described medical balloons made from LCP blends. U.S. Patent No. 5,389,314 to Wang discloses an inflatable medical device which has a plurality of longitudinally oriented conduits which extend through out the length of the device. The device may be formed by co-extruding two dissimilar plastic materials. The first material form defining a discrete phase which forms fibers and the other material or continuous phase which forms the remaining balloon material. After extrusion the discrete phase is withdrawn from the continuous phase, leaving the continuous phase with a plurality of conduits therethrough.

From EP 0 934 755 A2 a fiber reinforced balloon and catheter is known. In the known device, medical tubing involves combining a plurality of strengthening fibers with a polymer matrix. During extrusion the fibers become embedded in the matrix, and each fiber is forced into having an aspect ratio greater than about 50 to 1. The result is a tubing wherein the embedded fibers are substantially parallel to each other and are oriented substantially parallel to the lumen of the tubing.

WO 00/50105 discloses a medical device, at least a portion of which is composed of a polymeric material in which the polymeric material is a melt blend product of at least two different thermoplastic polymers. The portion of the device made from the melt blend may be a catheter body segment or a balloon for a catheter.

BRIEF SUMMARY OF THE INVENTION

The present invention is directed generally to medical balloons which expand only to a predetermined extent, and which have minimal longitudinal and/or minimal radial growth during expansion. Specifically, the invention is directed to a stent delivery balloon composed of a micro-composite material which includes a longitudinal fibril structure that is either parallel to the longitudinal axis of the balloon structure, or that is diagonal to the longitudinal axis at the molecular level of the balloon. The orientation of the fibril structure can limit longitudinal expansion of the balloon and allow the balloon to expand radially as desired, but minimally, or not at all in the longitudinal direction if the fibrils are parallel to the balloon axis, or when the fibrils are oriented diagonally about the axis, can limit both longitudinal and radial expansion of the balloon when inflated.

The micro-composite material is made up of a combination of a fibril component, a semi-compliant balloon material which acts as a matrix, and optionally a compatibilizer material which may act to create a less distinctive phase boundary between the fibril and matrix components, but which does not solubilize the LCP polymer in the matrix at human body temperature.

The present invention provides for a balloon which utilizes LCP materials or other oriented materials such as PET, in combination with a thermoplastic elastomer matrix and an optional compatibilizer to form a micro-composite material. The present micro-composite material is suitable for construction balloons which exhibit minimal or no longitudinal growth during balloon expansion but which expands as desired in the radial direction, or the present micro-composite material is suitable for construction of balloons that exhibit minimal expansion both in the longitudinal and radial directions.

BRIEF DESCRIPTION OF THE SEVERAL VIEWS OF THE DRAWINGS

A detailed description of the invention is hereinafter described with specific reference being made to the drawings in which:

FIG. 1 is a schematic representation of side view of a tubular parison used to produce a balloon of the invention from a the micro-fiber composite material;

FIG. 2 is a schematic side view of a medical device delivery balloon constructed from micro-composite material shown at nominal diameter wherein the fibril component is oriented parallel to the longitudinal balloon axis.

FIG. 3 is a view of the medical device delivery balloon shown in Fig. 2 in an inflated state at a pressure higher which causes radial growth of the balloon;

FIG. 4 is a cross-sectional view of a tubular parison for producing balloon of an alternative embodiment of the invention; and

FIG. 5 is a perspective view of the embodiment shown in FIG. 4.

FIG. 6 is perspective view of a dilatation balloon preform in a tubular parison form constructed from micro-composite material wherein the inner and outer fibril components have been oriented diagonally to the longitudinal axis of the tubular preform and in crossing relationship relative to each other by use of a counter-rotating extrusion die.

FIG. 7 is another perspective view of only the outer surface of a dilatation balloon preform constructed from micro-composite material wherein the fibril component is oriented diagonally to the longitudinal axis of the tubular preform by use of a rotating die.

FIG. 8 is a schematic side-view of a blow molded dilatation balloon constructed from micro-composite material depicting the fibril component oriented diagonally to the longitudinal axis of the balloon.

## DETAILED DESCRIPTION OF THE INVENTION

While this invention may be embodied in many different forms, there are shown in the drawings and described in detail herein specific preferred embodiments of the invention. The present disclosure is an exemplification of the principles of the invention and is not intended to limit the invention to the particular embodiments illustrated.

As noted above, the present invention relates to medical catheters which have one or more balloon portions constructed from a specially configured micro-composite material. The particular micro-composite material and configuration provides physical properties which allow a balloon to expand radially to a predetermined extent, but which allow only minimal, or more preferably, no longitudinal growth during expansion. The micro-composite material includes a longitudinal fibril component which exhibits micro-fibers at the molecular level in combination with a matrix of any semi-compliant balloon material. Depending on the specific fibril component, as well as the method of extrusion utilized to extrude the balloon material, the micro-fibers may be randomly scattered through out the balloon material or may be precisely spaced about the balloon and extending through the entire balloon length. The fibril structure is oriented or directed in the longitudinal direction of the balloon providing the balloon with desirable radial expansion characteristics and minimal longitudinal growth when the balloon is inflated.

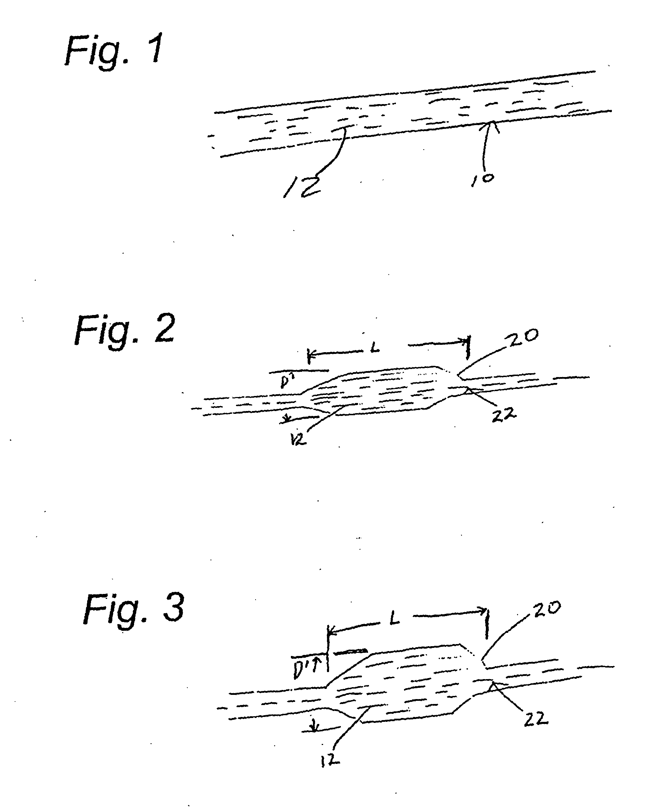

As shown in Fig. 1, the balloons of the invention may be made from tubular parisons 10 of the micro-composite material, having a fibril component which exhibits micro-fibers 12 uniformly oriented in a predetermined direction. In a preferred embodiment shown in Fig. 2, the micro-composite is formed into a balloon 20 from a parison 10 by a conventional balloon blowing process. Balloon 20 has a diameter D and a length L. Micro-fibers 12 are oriented along and about the longitudinal axis 22 of the balloon at the molecular level. The fibril component may be any rigid-rod or semi-rigid-rod thermoplastic material which comprises 0.1 to 20 percent, and more preferably from 0.5 to 15 percent by weight of the micro-composite material.

Examples of suitable materials which could be utilized as the fibril component include: liquid crystal polymers such as VECTRA® LKX 1107, 1111, polyetheretherketone (PEEK) material, and PPS. Other materials may also be utilized as the fibril component of the present invention. Such substances include aromatic nylon, rigid polyurethane, polyester, copolyester, polyester blends, polyester/polyurethane blends, PEEK, PPS, fluoropolymer and so on.

To form the micro-composite material, the fibril component is preferably combined with a semi-compliant thermoplastic polymer material in a melt blend which at least partially phase separates upon cooling. Under appropriate conditions the phase separated material will form fibrils or micro-fiber 12 embedded in a matrix of the semi-compliant thermoplastic polymer, oriented substantially parallel to the longitudinal axis of the extruded tubing. The micro-composite material suitably employs an amount of semi-compliant polymer matrix component from 50 to 99.9 percent by weight, preferably from 85 to 99.5 percent.

Some examples of suitable materials which may be utilized as the matrix component are polyamide-polyester block copolymers, namely the polyamide/polyether/polyesters PEBAX<> 6333, 7033 and 7233; also polyester-polyether block copolymer such as ARNITEL<> 540.

As previously described, the present invention achieves the desired balloon expansion characteristics as a result of forming a balloon composed of a micro-composite material. The micro-composite material balloon is formed by coextrusion of a melt blend of LCP or other orientable material, the matrix component, and optionally a compatibilizer. A dual extrusion process utilizing two extruders may also be used to form the desired tube. In the case where LCP is used as the fibril component, the longitudinally oriented fibers are formed by subjecting the blend material to a relatively high extrudate puller speed. The high speed of the puller will subject the blend material to a shearing force which causes a material such as LCP to elongate and form fibers. If the LCP is not subjected to a high shearing force, the LCP will form droplet shaped deposits which provide minimal or no longitudinal stabilization.

If, during extrusion, relative rotation of the mandrel and die is avoided, the fibrils will adopt an orientation substantially parallel to the longitudinal axis. It the die and mandrel are relatively rotated, e/g. by rotation of one or the other or both, the orientation of the fibrils will be helically about the axis.

A balloon which has an LCP fibril component tends to have individual fibers spread randomly throughout the balloon material. The individual LCP fibers will typically be between 0.1 μm (micron) to 1 μm (micron) in diameter.

If the various components utilized to form the micro-composite material are incompatible to a substantial degree, phase separation may be so efficient that slippage between phases might occur during balloon expansion thereby reducing the longitudinal restriction effect of the fibrils. To prevent such occurrences a compatibilizer may also be desirable for the purpose of enhancing the homogeneity of the melt blend prior to extrusion and cooling. A compatibilizer material may be added to the pre-extruded melt blend material to create a less distinctive phase boundary between the fibril and matrix components. The compatibilizer may be for instance a block copolymer comprising a block which is structurally similar or otherwise is soluble in the matrix polymer and a block which is structurally similar or otherwise soluble with the fibril component. An example of a suitable is the melt compatibilizer disclosed in application 08/926,905. Such a compatibilizer may be employed in an amount from 0 to 30 weight percent.

The balloon 20, shown in Fig. 2 at nominal diameter, is shown in Fig. 3 inflated at a higher pressure which provides radial expansion to a new, larger diameter D'. In the most preferred embodiment, the micro-composite material 10 allows balloon 20 to obtain semi-compliant expansion in the radial direction while negating balloon expansion in the longitudinal direction during inflation (balloon length L is substantially unchanged in Fig. 3). Depending on the precise mixture and type of matrix and fibril components used, other embodiments of the present invention may provide for balloons with varying degrees and types of radial expansion while also reducing longitudinal expansion by varying degrees.

If substances less prone to phase separation from the matrix material are desired to be used, an appropriately shaped die may be used in the extrusion process to provide individually extruded fibers evenly around the tube circumference, for instance in the manner of US 5,389,314 except that the fiber material is selected to adhere to the matrix material and a high line speed is used to provide a microscopic fiber diameter. For such an embodiment, the individual non-LCP fibers will typically be between 10 - 12 μm (microns) in diameter and may also extend through the entire length of the balloon in chain or cores.

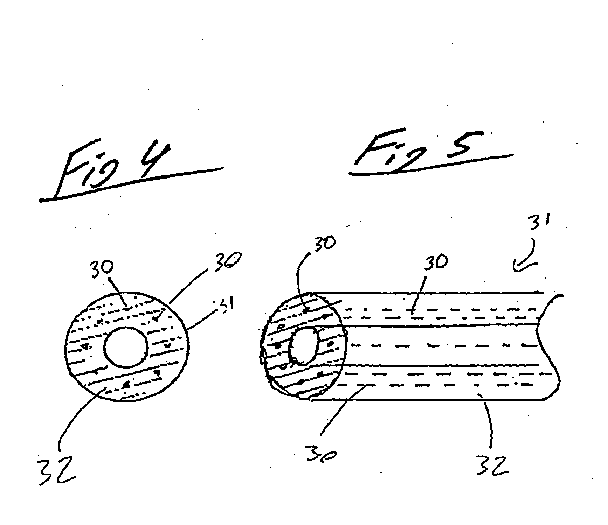

This embodiment is depicted by the tubular parisons in Figs. 4 and 5. As shown, cores 30 are suspended through out the parison 31 in a matrix 32 which may be composed of any material suitable for constructing a semi-compliant balloon as have been described above. The cores 30 are composed of a material which has a more limited ability to stretch than the matrix material, and when the cores are collectively oriented in the same direction, the structures exhibit an increased longitudinal stability when inflated beyond initial or nominal diameter.

In selecting appropriate materials for the fibrils of cores 30 and matrix 32 it is important to select materials which provide adequate adhesion to one another. If adhesion is insufficient between the cores 30 and the surrounding matrix 32 longitudinal growth of the balloon produced from parison 31 will not be restricted as the more expansive matrix material will slip past the individual cores. A further important attribute of the cores 30 is the bulk elongation of the material when oriented as described above. The bulk elongation of the cores 30 should be within the range of 50%-150%. In order to avoid core breakage prior to balloon bursting, it is desirable to the present invention that if the material from which the cores are constructed exhibit a higher tensile strength than the material of which the matrix is constructed.

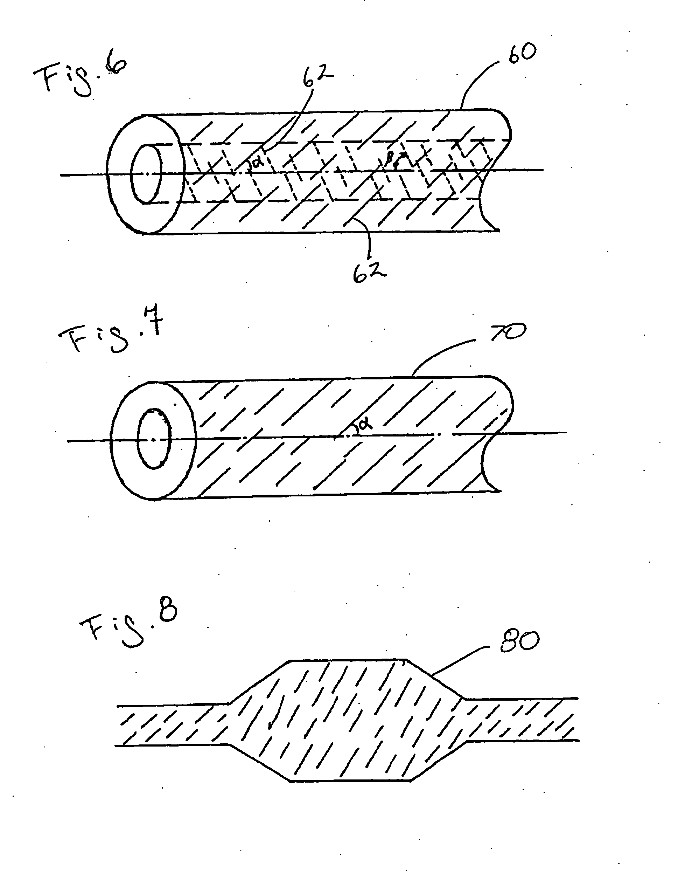

Figures 6-8 pertain to alternative embodiments in which the fibers of the balloon are orientated diagonally relative to the longitudinal axis of the balloon. In Figure 6 there is depicted a parison 60 for a balloon in which, in addition to using a high puller speed during extrusion, a counter rotating die was used. The counter rotating die has a mandrel which rotates in one direction and a concentric outer die which rotates in the opposite direction, the parison is extruded through the space between the two. The resulting parison has fibers 62 orientated diagonally to the parison axis 64 in one direction at the outside surface (angle α) and changing gradually as one passes through the material in a direction transverse to the axis 64 to a second direction (angle β) at the inside surface, the angles determined by outer die/mandrel rotation speeds and puller speed. If one or the other of the outer die or the mandrel are held stationary while the other is rotated, angle α or angle β may be parallel to the axis 64.

In Figure 7 there is depicted a parison 70, having diagonally oriented fibers formed by relative rotation of the die and puller. For instance only the outer die or mandrel may be rotated so that the fibers become orientated at angle α throughout the entire thickness of the parison.

Figure 8 depicts the outer surface orientation of a balloon 80 made from a parison of either Fig. 6 or Fig 7. In the balloon body the fibers retain an angular orientation relative to the balloon axis and provide resistance to both longitudinal and radial expansion beyond the nominal or molded dimensions.

Based on the above description it should be understood that several different polymers with a wide range of characteristics may be used to form a longitudinal or longitudinal and radial stabilized balloon of the present invention. The following is an example of a balloon and its manufacturing parameters which was actually constructed in accordance with the present invention disclosure.

Example 1: a matrix component of Pebax 7033 was mixed with a fibril component of LCP VECTRA LKX 1107 at the ratio of 95% to 5% respectively by weight.

The mixture was extruded at a rate of 0.5588 $\frac{m}{s}$ (110 feet/minute) line speed into tubing of 0.099 (0.039) (outer diameter) x 0.069 (0.027) (inner diameter) cm (inch). A 3.5 mm balloon was formed from the resulting tubing by radial expansion at 110 degrees Celsius with blowing pressure of 24.1325 bar (350 psi). The balloon with double wall thickness of 0.003556 cm (0.0014 inch) was inflated from 4.0532 bar (4 atm) to 13.1729 bar (13 atm) at 1.0133 bar (1 atm) increment and no measurable balloon length change was observed.

Examples of balloons are listed below:

1. A dimensionally stable polymer balloon having a longitudinal axis and composed of a micro-composite material, the micro-composite material comprising a polymer matrix component and a polymer fibril component distributed in the polymer matrix component, the fibril component having micro-fibers oriented substantially parallel or diagonally to the longitudinal axis of the balloon.

2. The dimensionally stable balloon of number 1 mounted on a catheter.

3. The dimensionally stable balloon of number 1, wherein said fibril component comprises about 20% by weight or less but greater than about 0.1 % of said microcomposite material.

4. The dimensionally stable balloon of number 1, wherein the micro-composite material further comprises a compatibilizer component.

5. The dimensionally stable balloon of number 4 wherein said compatibilizer is a block copolymer

6. The dimensionally stable balloon of number 1, wherein the fibril component is composed of rigid-rod thermoplastic material.

7. The dimensionally stable balloon of number1, wherein the fibril component is composed of semi-rigid-rod thermoplastic material.

8. The dimensionally stable balloon of number 1, wherein the fibril component is composed of liquid crystal polymer material.

9. The dimensionally stable balloon of number 1, wherein the matrix component is a semi-compliant thermoplastic polymer.

10. The dimensionally stable balloon of number 1, wherein the micro-fibers are oriented substantially parallel to the longitudinal axis of the balloon.

11. The dimensionally stable balloon of number 1, wherein the micro-fibers are oriented diagonally to the longitudinal axis of the balloon.

12. The dimensionally stable balloon of number 1, wherein the orientation of the microfibers relative to the longitudinal axis of the balloon changes through the balloon material in a direction transverse to said longitudinal axis.

13. A method of forming a balloon composed of a micro-composite material comprising the steps of :

(a) melt blending a matrix component and a fibril component, wherein the mixture comprises less than about 15 percent by weight but greater than about 0.5 percent by weight of said fibril component, and the matrix component comprises less than about 99.5 percent by weight but greater than about 85 percent by weight of said matrix component;

(b) forming the melt blended mixture into tubing by extrusion in a manner which orients the fibril component along the longitudinal axis of the tubing; and

(c) forming the balloon by radial expansion of a segment of the tubing.

14. The method of number 13 further comprising adding a compatibilizer to the melt blended mixture.

15. An inflatable medical balloon having a determined pre-inflation length, restricted longitudinal or radial expansion characteristics, a circumference and a longitudinal axis comprising:

a matrix material, said matrix material characterized as being semi-compliant; and

having a plurality of cores therethrough, said cores being evenly distributed about the circumference of the balloon and being composed of one or more materials which are characterized as being stronger than the matrix material and having a bulk elongation less than the matrix material when the one or more materials are oriented in the direction of the longitudinal axis, and the matrix material and the core material operatively adhering to one another.

16. The medical balloon of number 15 wherein the bulk elongation of the one or more cores material is between 50% and 150%.

## Claims

1. An inflatable medical balloon having a determined pre-inflation length, restricted longitudinal or radial expansion characteristics, a circumference and a longitudinal axis comprising:

● a matrix material, said matrix material **characterized** as being semi-compliant;

● and having a plurality of cores therethrough, said cores being evenly distributed about the circumference of the balloon and being composed of one or more materials which are **characterized** as having a higher tensile strength than the matrix material and having a bulk elongation less than the matrix material when the one or more materials are oriented in the direction of the longitudinal axis, and the matrix material and the core material operatively adhering to one another.

2. The medical balloon of claim 1, wherein the bulk elongation of the cores is between 50 % and 150 %.

**Patentansprüche**

1. Aufblasbarer medizinischer Ballon mit einer vorbestimmten Länge vor dem Aufblasen, begrenzten Aufweitungsmerkmalen in Längs- oder Radialrichtung, einem Umfang und einer Längsachse, umfassend:

• ein Matrixmaterial, wobei das Matrixmaterial **dadurch gekennzeichnet ist**, halbverträglich zu sein;

• und mit einer Vielzahl von Kernen durch es hindurch, wobei die Kerne gleichmäßig um den Umfang des Ballons verteilt sind und aus einem oder mehreren Materialien zusammengesetzt sind, welche **dadurch gekennzeichnet sind, dass** sie eine höhere Zugfestigkeit als das Matrixmaterial aufweisen und eine geringere Volumendehnung als das Matrixmaterial haben, wenn das eine oder die mehreren Materialien in der Richtung der Längsachse orientiert sind, und das Matrixmaterial und das Kernmaterial operativ aneinander haften.

2. Medizinischer Ballon gemäß Anspruch 1, wobei die Volumendehnung der Kerne zwischen 50% und 150% ist.

**Revendications**

1. Ballonnet médical gonflable avec une longueur prédéterminée avant le gonflement, des caractéristiques d'expansion longitudinales ou radiales restreintes, une circonférence et un axe longitudinal, comportant:

• un matériau de matrice, ledit matériau de matrice étant **caractérisé** comme demi compatible ;

• et avec une pluralité de noyaux à travers de celui-ci, lesdits noyaux étant distribués de façon régulière sur la circonférence du ballonnet et étant composés d'un ou plusieurs matériaux qui sont **caractérisés** comme ayant une résistance à la tension plus haute que le matériau de matrice et comme ayant une dilatation de volume moins grande que le matériau de matrice lorsque l'un ou les plusieurs matériaux sont orientés dans la direction de l'axe longitudinal, et le matériau de matrice et le matériau des noyaux adhérent l'un à l'autre de façon opérative.

2. Ballonnet médical selon la revendication 1, dans lequel la dilatation de volume des noyaux est entre 50% et 150%.

## Fig. 1

## Fig. 2

## Fig. 3

Fig 4

Fig 5

Fig. 6

Fig. 7

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5702418 A
- US 5797877 A
- US 926905 A
- US 9818345 W
- US 09257677 B
- US 5389314 A, Wang
- EP 0934755 A2
- WO 0050105 A